# EUROPÄISCHE PATENTANMELDUNG

(11) **EP 4 248 956 A2**
(43) Veröffentlichungstag der Anmeldung: **27.09.2023**
(21) Anmeldenummer: 23162192.1
(22) Anmeldetag: 15.03.2023
(51) Int. Cl.: A61K 31/05, A61K 35/742, A61K 9/00, A61K 8/99

(54) **PHARMAZEUTISCHE ZUSAMMENSETZUNG MIT CANNABIDIOL**

(71) Anmelder: Patentpool Target GmbH, 80331 München (DE)
(72) Erfinder: Borrmann, Elmar, 80331 Muenchen (DE)
(74) Vertreter: Reich, Jochen

(57) **Zusammenfassung**

Die vorliegende Erfindung betrifft eine Zusammensetzung aufweisend Cannabidiol und Bacillus Subtilis. Werden beide Substanzen zusammen verwendet, haben sie überraschenderweise synergistische Wirkungen und verstärken sich gegenseitig. Cannabidiol hat antibakterielle Eigenschaften, welche mit Bacillus Subtilis über die reine Kombination hinaus in unterschiedlichen Anwendungsbereichen noch vorteilhaft verstärkt werden. Darüber hinaus betrifft die Erfindung ein entsprechend eingerichtetes Verfahren zur Herstellung der Zusammensetzung sowie ein Nahrungsergänzungsmittel und ein kosmetisches Produkt.

## Beschreibung

Die vorliegende Erfindung betrifft eine Zusammensetzung aufweisend Cannabidiol und Bacillus Subtilis. Werden beide Substanzen zusammen verwendet, haben sie überraschenderweise synergistische Wirkungen und verstärken sich gegenseitig. Cannabidiol hat antibakterielle Eigenschaften, welche mit Bacillus Subtilis über die reine Kombination hinaus in unterschiedlichen Anwendungsbereichen noch vorteilhaft verstärkt werden. Darüber hinaus betrifft die Erfindung ein entsprechend eingerichtetes Verfahren zur Herstellung der Zusammensetzung sowie ein Nahrungsergänzungsmittel und ein kosmetisches Produkt.

Gemäß dem Stand der Technik kann eine Wirkung von Cannabidiol (CBD) durch verschiedene Methoden verbessert werden, wie der Erhöhung der Dosierung: Die Erhöhung der Dosis kann die Wirkung von Cannabidiol verstärken, wobei es angezeigt ist, mit einer niedrigen Dosis zu beginnen und sich allmählich zu steigern, um unerwünschte Nebenwirkungen zu vermeiden. Eine Verwendung von Vollspektrum- Cannabidiol -Produkten erhöht ebenfalls die Wirkung von Cannabidiol. Vollspektrum- Cannabidiol -Produkte enthalten neben Cannabidiol auch andere Cannabinoide, Terpene und Flavonoide, die in Kombination mit Cannabidiol synergistisch wirken und die Wirkung von CBD verstärken können. Es können auch liposomale Cannabidiol -Produkte verwendet werden. Liposomale Cannabidiol - Produkte sind mit Phospholipiden umhüllt und können aufgrund ihrer kleineren Größe und höheren Bioverfügbarkeit die Wirkung von Cannabidiol verstärken. Verwendung von Cannabidiol -Produkten in Kombination mit anderen natürlichen Substanzen erhöht ebenfalls die Wirkung. Cannabidiol kann in Kombination mit anderen natürlichen Substanzen wie Kurkuma, Ingwer oder Pfeffer verstärkt werden, da diese Substanzen die Bioverfügbarkeit von Cannabidiol verbessern und seine Wirkung verstärken können.

Es besteht im Stand der Technik ein Bedarf alternative Zusammensetzungen beziehungsweise Verfahren zu deren Herstellung zu schaffen, welche mit geringem technischem Aufwand zu schaffen sind. Von einer Erhöhung der Dosis von Cannabidiol soll aufgrund von Kosteneffizienz abgesehen werden. Cannabidiol ist in der Herstellung technisch aufwendig und soll in möglichst geringen Dosen verabreicht werden ohne die Wirkung abzuschwächen.

Es ist daher eine Aufgabe ein alternatives Verfahren bzw. eine alternative Zusammensetzung zu schaffen, welche die Wirkung von Cannabidiol erhöht. Diese Erhöhung der Wirkung soll in technischer Weise effizient erfolgen und es soll vermieden werden, dass einfach eine höhere Menge des aufwendig herzustellenden Cannabidiols verabreicht wird. Darüber hinaus soll ein entsprechendes Nahrungsergänzungsmittel sowie ein kosmetisches Produkt vorgeschlagen werden.

Die Aufgabe wird gelöst durch eine Zusammensetzung mit den Merkmalen gemäß Patentanspruch 1. Weitere vorteilhafte Ausgestaltungen sind in den Unteransprüchen angegeben.

Demgemäß wird eine pharmazeutische Zusammensetzung vorgeschlagen aufweisend Bacillus Subtilis und Cannabidiol.

CBD (Cannabidiol) und Bacillus Subtilis sind zwei separate Substanzen, die bisher getrennt voneinander verwendet werden. Cannabidiol ist eine Verbindung, die aus der Hanfpflanze gewonnen wird und in verschiedenen Formen erhältlich ist, einschließlich Kapseln und Topika. Bacillus Subtilis ist eine Art von Bakterium, das in der Umwelt vorkommt und in der Lebensmittelindustrie sowie in der Landwirtschaft verwendet wird.

Es gibt im Stand der Technik keine direkte Kombination von Cannabidiol und Bacillus Subtilis, da es sich um verschiedene Substanzen handelt, die in unterschiedlichen Anwendungen eingesetzt werden. Diese wird erfindungsgemäß vorgeschlagen, da sie in einigen Fällen gemeinsam eingesetzt werden können, um synergetische Vorteile zu erzielen.

Zudem wird erfindungsgemäß der Nachteil überwunden, dass im Stand der Technik dem Bacillus Subtilis zusätzlich Silizium beigemengt werden muss. Erfindungsgemäß wurde überraschenderweise erkannt, dass dies nicht notwendig ist und somit Silizium eingespart werden kann.

Einige der potenziellen Vorteile von Cannabidiol umfassen die Reduzierung von Schmerzen und Entzündungen, die Verbesserung von Schlaf und Stimmung sowie die Unterstützung des Immunsystems. Bacillus Subtilis hingegen kann dazu beitragen, das Verdauungssystem zu unterstützen, indem es den Darmtrakt von schädlichen Bakterien befreit und das Wachstum von gesunden Bakterien fördert.

Wenn beide Substanzen zusammen verwendet werden, haben sie überraschenderweise synergistische Wirkungen und verstärken sich gegenseitig. Beispielsweise kann Bacillus Subtilis die Aufnahme von CBD durch den Körper verbessern und die Stärkung des Immunsystems unterstützen, während CBD unter anderem dazu beiträgt, Entzündungen im Darm zu reduzieren und das Verdauungssystem zu beruhigen. Zum Beispiel bekämpft Bacillus Subtilis Bakterien, die schlecht für die Darmflora oder die Lebensmittelverträglichkeit sind, indem er ihre Zellwände zerstört. Außerdem trägt er dazu bei, dass Nährstoffe gut ins Blut aufgenommen werden können. Dies ist gerade bei Cannabidiol vorteilhaft.

Die vorgeschlagene Zusammensetzung kann zur Behandlung von Verdauungsstörungen oder zur Unterstützung der Darmgesundheit bei einem Säugetier, bevorzug beim Menschen, durch die Verabreichung einer pharmazeutischen Zusammensetzung, die eine wirksame Menge von Cannabidiol (CBD) und Bacillus Subtilis enthält dienen, wobei die Kombination von Cannabidiol und Bacillus Subtilis eine synergistische Wirkungen aufweist, um das Wachstum von nützlichen Darmbakterien zu fördern, das Wachstum von schädlichen Bakterien zu hemmen, Entzündungen und Schäden im Darm zu verringern und das Immunsystem zu stärken. Darüber werden die Wirkungen von Cannabidiol durch den Bacillus Subtilis aufrechterhalten und Cannabidiol wird in besonders großer Menge absorbiert.

Cannabidiol ist aufwendig in der Herstellung und somit eignet sich gerade der Bacillus Subtilis um die Aufnahme des Cannabidiols zu verbessern. Dies hat den technischen Effekt, dass Cannabidiol lediglich in geringeren Dosen aufgenommen werden muss, da dieses in höherer Konzentration absorbiert wird.

Überraschenderweise eignet sich der Bacillus Subtilis besonders bei einem Einsatz von Cannabidiol, denn er ist vollkommen ungefährlich. Im Gegensatz zu anderen Bakterien aus dem Stand der Technik löst Bacillus Subtilis keine Krankheiten aus, sondern kann sogar bei der Bekämpfung von bakteriellen Infektionen helfen. Nicht alle Stämme von Bacillus Subtilis können allerdings Antibiotika synthetisieren.

Vorliegend wird eine pharmazeutische Zusammensetzung vorgeschlagen, wobei pharmazeutisch klarstellend verstanden wird und somit optional ist. Überraschenderweise wurde zudem erkannt, dass Cannabidiol nicht als Öl verwendet werden muss.

Bacillus Subtilis ist ein Bakterium, das natürlicherweise im Verdauungstrakt von Tieren und Menschen vorkommt. Es ist auch als probiotischer Organismus bekannt, was bedeutet, dass es eine positive Wirkung auf die Gesundheit des Verdauungstrakts haben kann. Einige der möglichen synergistische Effekte von Bacillus Subtilis in Kombination mit Cannabidiol sind:
- Unterstützung der Verdauung: Bacillus Subtilis kann dabei helfen, das Gleichgewicht der Darmflora wiederherzustellen und die Verdauung zu verbessern. Es kann auch dazu beitragen, Nahrungsmittelallergien und - unverträglichkeiten zu reduzieren.
- Unterstützung des Immunsystems: Bacillus Subtilis kann das Immunsystem in Kombination mit Cannabidiol stärken, indem es die Produktion von Immunzellen und Antikörpern fördert, die Infektionen bekämpfen.
- Schutz vor schädlichen Bakterien: Bacillus Subtilis kann dazu beitragen, schädliche Bakterien im Verdauungstrakt zu hemmen und das Wachstum von nützlichen Bakterien zu fördern, was die Gesundheit des Verdauungstrakts verbessert.
- Unterstützung bei der Gewichtsreduktion: Cannabidiol wird eine appetitzügelnde Eigenschaft nachgesagt. Bacillus Subtilis kann helfen, den Stoffwechsel zu verbessern und den Körper bei der Fettverbrennung zu unterstützen, was zu einer Gewichtsreduktion führen kann. Überraschenderweise wurde erfindungsgemäß erkannt, dass sich beide Substanzen in ihrer Wirkung über die reine Kombination hinaus noch verstärken.
- Unterstützung bei der Bekämpfung von Durchfall: Bacillus Subtilis kann bei der Bekämpfung von Durchfall helfen, indem es das Wachstum von schädlichen Bakterien im Verdauungstrakt hemmt und die Darmflora wiederherstellt, was Cannabidiol unterstützt.

Gemäß einem Aspekt der vorliegenden Erfindung weist die Zusammensetzung Trägermaterialien und/ oder Füllstoffe auf. Dies hat den Vorteil, dass die Verabreichung in geeignetem Volumen stattfinden kann und die Zusammensetzung mit weiteren Stoffen kombiniert werden kann.

Gemäß einem weiteren Aspekt der vorliegenden Erfindung macht das Cannabidiol mindestens 60% der Zusammensetzung aus. Dies hat den Vorteil, dass ein überwiegender Anteil von Cannabidiol bevorzugt von 80 oder 90% verwendet werden kann. Der Bacillus Subtilis wird in relativ geringen Mengen verabreicht.

Gemäß einem weiteren Aspekt der vorliegenden Erfindung macht das Cannabidiol 12, 25, 30 oder 50% der Zusammensetzung aus.

Gemäß einem weiteren Aspekt der vorliegenden Erfindung weist die Zusammensetzung Ballaststoffe, Vitamine und/ oder weitere Probiotika auf. Dies hat den Vorteil, dass weitere Effekte erzeugt werden können und insbesondere die Verwendung von weiteren Probiotika stellt die Wirksamkeit des Bacillus Subtilis sicher. Auf diese Weise wird die Wirksamkeit des Cannabidiols nochmals verstärkt, da es besser im menschlichen Körper absorbiert wird.

Gemäß einem weiteren Aspekt der vorliegenden Erfindung weist die pharmazeutische Zusammensetzung eine höhere Konzentration von Cannabidiol als von Bacillus Subtilis auf. Dies hat den Vorteil, dass ein vorteilhaftes Mischverhältnis entsteht und vor allem das Cannabidiol verabreicht wird, wobei der Bacillus Subtilis lediglich unterstützend eingesetzt wird.

Gemäß einem weiteren Aspekt der vorliegenden Erfindung ist der Bacillus Subtilis aus einem der Stämme LA13030, CU1, HU58, DSM 33619, DE 111, DSM 15544, Rosell-179 und/ oder PXN 21 gewählt. Dies hat den Vorteil, dass die Bacillus Subtilis-Stämme ausgewählt werden, welche eine verbesserte Darmkolonisation und Überlebensfähigkeit im Gastrointestinaltrakt ermöglichen. Diese haben sich in Kombination mit Cannabidiol als besonders wirksam erwiesen.

Gemäß einem weiteren Aspekt der vorliegenden Erfindung weist die Zusammensetzung Lebensmittelzusatzstoffe auf. Dies hat den Vorteil, dass die Verabreichung für den Endverbraucher variiert werden kann und in geeigneter und ansprechender Weise angeboten werden kann.

Die Aufgabe wird auch gelöst durch ein Verfahren zur Herstellung einer pharmazeutischen Zusammensetzung, aufweisend ein Bereitstellen von Bacillus Subtilis; ein Bereitstellen von Cannabidiol; und Vermengen des Bacillus Subtilis und Cannabidiol.

Die Aufgabe wird auch gelöst durch ein Nahrungsergänzungsmittel und ein kosmetisches Produkt.

Weitere Vorteile, Merkmale und Einzelheiten der Erfindung ergeben sich aus der nachfolgenden Beschreibung, in der unter Bezugnahme auf die Zeichnungen Aspekte der Erfindung im Einzelnen beschrieben sind. Dabei können die in den Ansprüchen und in der Beschreibung erwähnten Merkmale jeweils einzeln für sich oder in beliebiger Kombination erfindungswesentlich sein. Ebenso können die vorstehend genannten und die hier weiter ausgeführten Merkmale je für sich oder zu mehreren in beliebigen Kombinationen Verwendung finden. In der Figur zeigt:

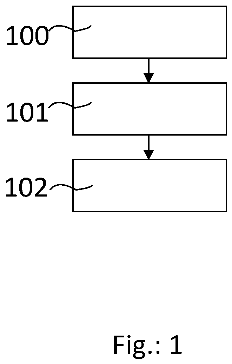

- Figur 1:: ein schematisches Ablaufdiagramm des vorgeschlagenen Verfahrens gemäß einem Aspekt der vorliegenden Erfindung.

Figur 1 zeigt in einem schematischen Ablaufdiagramm ein Verfahren zur Herstellung einer pharmazeutischen Zusammensetzung, aufweisend ein Bereitstellen 100 von Bacillus Subtilis; ein Bereitstellen 101 von Cannabidiol; und ein Vermengen 102 des Bacillus Subtilis und Cannabidiol.

## Patentansprüche

1. Pharmazeutische Zusammensetzung, aufweisend:
- Bacillus Subtilis; und
- Cannabidiol.

2. Pharmazeutische Zusammensetzung nach Anspruch 1, **dadurch gekennzeichnet, dass** die Zusammensetzung Trägermaterialien und/ oder Füllstoffe aufweist.

3. Pharmazeutische Zusammensetzung nach Anspruch 1 oder 2, **dadurch gekennzeichnet, dass** das Cannabidiol mindestens 60% der Zusammensetzung ausmacht.

4. Pharmazeutische Zusammensetzung nach einem der vorhergehenden Ansprüche, **dadurch gekennzeichnet, dass** die Zusammensetzung Ballaststoffe, Vitamine und/ oder weitere Probiotika aufweist.

5. Pharmazeutische Zusammensetzung nach einem der vorhergehenden Ansprüche, **dadurch gekennzeichnet, dass** die pharmazeutische Zusammensetzung eine höhere Konzentration von Cannabidiol als von Bacillus Subtilis aufweist.

6. Pharmazeutische Zusammensetzung nach einem der vorhergehenden Ansprüche, **dadurch gekennzeichnet, dass** der Bacillus Subtilis aus einem der Stämme LA13030, CU1, HU58, DSM 33619, DE 111, DSM 15544, Rosell-179 und/ oder PXN 21 gewählt ist.

7. Pharmazeutische Zusammensetzung nach einem der vorhergehenden Ansprüche, **dadurch gekennzeichnet, dass** die Zusammensetzung Lebensmittelzusatzstoffe aufweist.

8. Verfahren zur Herstellung einer pharmazeutischen Zusammensetzung, aufweisend:
- Bereitstellen (100) von Bacillus Subtilis;
- Bereitstellen (101) von Cannabidiol; und
- Vermengen (102) des Bacillus Subtilis und Cannabidiol.

9. Nahrungsergänzungsmittel aufweisend eine pharmazeutische Zusammensetzung nach einem der Ansprüche 1 bis 7.

10. Nahrungsergänzungsmittel nach Anspruch 12, **dadurch gekennzeichnet, dass** dieses als Öl, ölige Lösung, Tee, Granulat, Tablette, Kapsel, Pastille, Kaugummi und/ oder Bonbon vorliegt.

11. Kosmetisches Produkt aufweisend eine pharmazeutische Zusammensetzung nach einem der Ansprüche 1 bis 7.

12. Kosmetisches Produkt nach Anspruch 11, **dadurch gekennzeichnet, dass** dies als Creme vorliegt.
